(19) Europäisches Patentamt
European Patent Office
Office européen des brevets

(11) **EP 2 040 776 B1**

(12) **EUROPÄISCHE PATENTSCHRIFT**

(45) Veröffentlichungstag und Bekanntmachung des Hinweises auf die Patenterteilung:
**04.09.2013 Patentblatt 2013/36**

(21) Anmeldenummer: **07765160.2**

(22) Anmeldetag: **11.07.2007**

(51) Int Cl.:
***A61M 1/36*** *(2006.01)*

(86) Internationale Anmeldenummer:
**PCT/EP2007/006140**

(87) Internationale Veröffentlichungsnummer:
**WO 2008/006559 (17.01.2008 Gazette 2008/03)**

(54) **VERFAHREN UND VORRICHTUNG ZUR ÜBERWACHUNG EINES EXTRAKORPORALEN BLUTKREISLAUFS**

METHOD AND DEVICE FOR MONITORING AN EXTRACORPOREAL BLOOD CIRCUIT

PROCÉDÉ ET DISPOSITIF DE SURVEILLANCE D'UN CIRCUIT DE CIRCULATION SANGUINE EXTRACORPOREL

(84) Benannte Vertragsstaaten:
**AT BE BG CH CY CZ DE DK EE ES FI FR GB GR HU IE IS IT LI LT LU LV MC MT NL PL PT RO SE SI SK TR**

(30) Priorität: **14.07.2006 DE 102006032815**

(43) Veröffentlichungstag der Anmeldung:
**01.04.2009 Patentblatt 2009/14**

(73) Patentinhaber: **Fresenius Medical Care Deutschland GmbH**
**61352 Bad Homburg v.d.H. (DE)**

(72) Erfinder: **KLEINEKOFORT, Wolfgang**
**65779 Kelkheim (DE)**

(74) Vertreter: **Oppermann, Frank et al**
**OANDO Oppermann & Oppermann LLP**
**John-F.-Kennedy-Straße 4**
**65189 Wiesbaden (DE)**

(56) Entgegenhaltungen:
**EP-A- 1 584 339        WO-A-03/002174**
**WO-A-03/074109**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

**Beschreibung**

Die Erfindung betrifft ein Verfahren zur Überwachung eines extrakorporalen Blutkreislaufs während einer extrakorporalen Blutbehandlung, insbesondere einer chronischen Blutreinigungstherapie, beispielsweise während einer Hämodialyse, Hämofiltration oder Hämodiafiltration, und eine Vorrichtung zur extrakorporalen Blutbehandlung, insbesondere zur Hämodialyse, Hämofiltration oder Hämodiafiltration, mit einer Einrichtung zur Überwachung des extrakorporalen Blutkreislaufs.

Bei den bekannten Methoden der chronischen Blutreinigungstherapie, beispielsweise der Hämodialyse, Hämofiltration oder Hämodiafiltration wird Blut des Patienten über einen extrakorporalen Blutkreislauf geleitet, der in der Regel mit einem Schutzsystem ausgestattet ist, das permanent den arteriellen und venösen Druck innerhalb des Kreislaufs überwacht. Zweck der Drucküberwachung ist die Detektion von verschiedenen Komplikationen, die während der extrakorporalen Blutbehandlung auftreten können. Zu den möglichen Therapiekomplikationen zählen ein fehlerhafter Gefäßzugang, der beispielsweise auf eine Diskonnektion der Kanüle oder das Ansaugen der Kanüle zurückzuführen ist, ein auftretender Blutverlust auf Grund des Herausrutschens der Kanüle oder einer Leckage, ein Abknicken des Blutschlauches oder eine Koagulation im Blutschlauch.

Die bekannten Schutzsysteme sehen bei der Feststellung eines fehlerhaften Zustandes im extrakorporalen Blutkreislauf im Allgemeinen vor, die Blutpumpe zu stoppen, die Schlauchklemme in der venösen Blutleitung zu schließen sowie ein akustisches und/oder optisches Warnsignal auszulösen. Hierdurch wird die Blutbehandlungsvorrichtung in einen für den Patienten sicheren Zustand gebracht, der allerdings zu einer Unterbrechung der Therapie führt.

Es sind verschiedene Alarmsysteme bekannt, mit denen sich fehlerhafte Zustände im extrakorporalen Blutkreislauf detektieren lassen. Im Allgemeinen basieren die Alarmsysteme darauf, den Druck im arteriellen und venösen Zweig des extrakorporalen Kreislaufs zu überwachen. Auf einen fehlerhaften Zustand schließen die bekannten Alarmsystem beim Über- oder Unterschreiten vorab festgelegter Grenzwerte. In der Regel hängt die Entscheidung über den fehlerhaften Zustand nur davon ab, ob der aktuell gemessene Druck innerhalb eines vorgegebenen Bereichs liegt oder nicht.

Die auf der Druckmessung basierenden Alarmsysteme sprechen im Allgemeinen an, wenn der arterielle und/oder venöse Druck im extrakorporalen Kreislauf einen Schwellenwert über- oder unterschreitet, der von dem Klinikpersonal zwischen +/- 20 und +/- 60 Torr variiert werden kann. Alarmsysteme mit einem Vergleich der Druckwerte mit Schwellwerten haben den Vorteil, dass sie schnell einstellbar sind. Die Druckgrenzwerte, bei deren Über- oder Unterschreitung Alarm ausgelöst wird, können bei den bekannten Systemen im Allgemeinen von Hand eingegeben werden. Diese Grenzwerte sind sowohl von dem Patienten als auch der Behandlungsart abhängig und werden zu Beginn jeder Behandlung individuell festgelegt.

Bei einem großem Schwellenabstand ergibt sich zwar der Vorteil einer geringen Fehlalarmrate, allerdings führen große Schwellenabstände zu einer Zeitverzögerung zwischen der Feststellung eines patientenkritischen Zustands und dem Ansprechen des Alarmsystems. Zusätzlich besteht die Gefahr, dass ein patientenkritischer Zustand nicht mehr sicher detektiert werden kann. Ein kleiner Schwellenabstand hingegen trägt zwar zur Erhöhung der Patientensicherheit bei, führt jedoch zwangsläufig zu einer erhöhten Fehlalarmrate, die zu einer Desensibilisierung des Überwachungspersonals oder sogar zu einer manuellen Alarmdeaktivierung führen kann. Die bekannten Alarmsysteme, bei denen das Über- und/oder Unterschreiten eines Schwellwertes überwacht wird, haben eine Fehlalarmrate von 70-99,5% in Abhängigkeit von der zu überwachenden physiologischen Größe (Wiklund L, Hök B, Stähl K, Jordeby-Jönsson A. Postanaesthesia monitoring revisited: Frequency of true and false alarms from different monitoring devices. J. Clin Anesth 1994; 6:182-188).

Die Hauptursache von druckbedingten Fehlalarmen sind beispielsweise Bewegungen des Patienten, Schwankungen des Blutdrucks des Patienten und Schwankungen der Viskosität des Blutes des Patienten.

Wenn der Patient seine vertikale Position verändert, beispielsweise der Patient höher oder niedriger gelegt wird, erhöht bzw. erniedrigt sich der extrakorporale Druck um ca. 0,75 Torr pro cm Höhenzu- bzw. Abnahme. Dieser Effekt führt beispielsweise dazu, dass arterieller und venöser Druck um ca. 10-20 Torr sinken, wenn sich ein Patient aus einer sitzenden Position in eine Schlafposition begibt. Da zwischen Punktionsstelle und Herz in sitzender Position ein Höhenunterschied besteht, ist der Fisteldruck im Sitzen höher als im Liegen.

Im Verlauf nächtlicher Dialysesitzungen, insbesondere bei der Heimdialyse, treten druckbedingte Fehlalarme meist genau dann auf, wenn der Patient in die Tiefschlafphase eintritt. Die durch Tiefschlaf bedingte Blutdruckabsenkung führt zu einer Verringerung des Drucks im gesamten Gefäßzugang. Über die arterielle und venöse Dialysekanüle führt der Blutdruckabfall zu einer symmetrischen Verringerung des arteriellen und venösen Drucks im extrakorporalen Kreislauf. Entsprechend führt ein Blutdruckanstieg zu einer Erhöhung des arteriellen und venösen Drucks.

Viskositätsschwankungen des Patientenblutes treten in der Regel dann auf, wenn dem Patienten während der Blutbehandlung infolge einer sogenannten Ultrafiltration Wasser entzogen wird. Bei einer Verringerung der Ultrafiltrationsrate hingegen sinkt die Viskosität des Patientenblutes, da aus dem extra- und intrazellulären Volumen Gewebe- und Zellwasser in das Blutvolumen gelangt.

Um Fehlalarme infolge von Viskositätsänderungen des Patientenblutes zu vermeiden, ist es bekannt, Algorithmen zur adaptiven Drifterkennung einzusetzen, die in festgelegten zeitlichen Abständen, die um den aktuellen arteriellen und venösen Druckwert angeordneten Alarmgrenzen zentrieren. Beispielsweise führt eine stetige Viskositätserhöhung des Patientenbluts zur symmetrischen Erhöhung des Flusswiderstands im extrakorporalen Kreislauf, wodurch der arterielle Druck abfällt und der venöse Druck ansteigt. Eine Viskositätsverringerung hingegen bewirkt das Gegenteil. Nachteilig ist, dass die bekannten Alarmsysteme mit Algorithmen zur adaptiven Drifterkennung aber nur diejenigen Alarme vermeiden können, die aus einer relativ langsamen Änderung des arteriellen und/oder venösen Drucks im extrakorporalen Kreislauf resultieren.

Die EP 1 584 339 A2 beschreibt eine Vorrichtung zur Überwachung eines Gefäßzugangs während einer Dialysebehandlung, bei der sowohl im arteriellen als auch im venösen Zweig des extrakorporalen Blutkreislaufs der Druck mittels Drucksensoren überwacht wird. Aus dem arteriellen und venösen Druck werden zwei für den Zustand des Gefäßzugangs charakteristische Werte in einer Recheneinheit berechnet, die in einer Auswerteinheit zur Erkennung eines fehlerhaften Gefäßzuganges ausgewertet werden. Das bekannte Überwachungssystem basiert auf der Berechnung von zwei den Zustand des Gefäßzuganges charakteristischen Werten, die mit zwei Schwellwerten verglichen werden, wobei auf einen fehlerhaften Gefäßzugang dann geschlossen wird, wenn sowohl der erste und zweite charakteristische Wert negativ als auch der erste charakteristische Wert kleiner als der erste Schwellwert und der zweite charakteristische Wert kleiner als der zweite Schwellwert sind.

Zur Berechnung des ersten charakteristischen Wertes wird die Differenz zwischen der Summe des venösen und arteriellen Drucks einer nachfolgenden Messung und der Summe des venösen und arteriellen Drucks einer vorhergehenden Messung ermittelt, während zur Berechnung des zweiten charakteristischen Wertes die Differenz zwischen der Differenz des venösen und arteriellen Drucks einer nachfolgenden Messung und der Differenz des venösen und arteriellen Drucks einer vorhergehenden Messung ermittelt wird, wobei diese Druckdifferenzen aufeinander folgender Messungen fortlaufend addiert werden. Folglich basiert die Überwachung des Gefäßzugangs auf dem Vergleich von Messwerten des arteriellen und venösen Drucks, die in vorhergehenden und nachfolgenden Messungen ermittelt worden sind. Allein auf Grund einer einzigen Messung zu einem bestimmten Zeitpunkt des arteriellen und venösen Drucks hingegen wird ein möglicher Fehlerzustand mit dem bekannten Algorithmus nicht erkannt.

Aus der WO 03/002174 A1 ist ein Verfahren bekannt, bei dem die Summe der Ableitungen des in der arteriellen Blutleitung gemessenen arteriellen Drucks und des in der venösen Blutleitung gemessenen venösen Drucks berechnet und die berechnete Summe der Druckwertänderungen mit einem vorgegebenen Grenzwert verglichen wird.

Aus der DE 101 59 620 C 1 ist eine Einrichtung zur Überwachung der Zufuhr von Substitutionsflüssigkeit während einer extrakorporalen Blutbehandlung bekannt, bei der nicht der Druck im extrakorporalen Blutkreislauf als solcher überwacht wird, sondern auf eine Störung der Substitutionsflüssigkeitszufuhr dann geschlossen wird, wenn die Amplitude der von der Substituatpumpe erzeugten Druckwellen einen vorgegebenen Grenzwert überschreitet.

Die US 2002/0190863 A1 beschreibt ein medizinisches Überwachungssystem, bei dem Fehlalarme dadurch ausgeschlossen werden sollen, dass die Zeitreihen zweier Messgrößen zur Auswertung herangezogen werden.

Der Erfindung liegt die Aufgabe zugrunde, ein Verfahren zur Überwachung eines extrakorporalen Blutkreislaufs während einer extrakorporalen Blutbehandlung anzugeben, mit dem die Anzahl von Fehlalarmen reduziert wird und das nur einen geringen apparativen Aufwand ohne zusätzliche Sensoren erfordert. Diese Aufgabe wird erfindungsgemäß mit den Merkmalen des Patentanspruchs 1 gelöst.

Eine weitere Aufgabe der Erfindung liegt darin, eine Vorrichtung zur extrakorporalen Blutbehandlung mit einer derartigen Einrichtung zur Überwachung des extrakorporalen Blutkreislaufs zu schaffen. Die Lösung dieser Aufgabe erfolgt erfindungsgemäß mit den Merkmalen des Patentanspruchs 7.

Vorteilhafte Ausführungsformen der Erfindung sind Gegenstand der Unteransprüche.

Das erfindungsgemäße Verfahren und die erfindungsgemäße Vorrichtung beruhen darauf, dass während der Blutbehandlung der arterielle Druck im arteriellen Zweig und der venöse Druck im venösen Zweig des extrakorporalen Kreislaufs gemessen wird, wobei fortlaufend die Summe des zu dem selben Zeitpunkt gemessenen arteriellen und venösen Drucks und die Differenz des zu dem selben Zeitpunkt gemessenen arteriellen und venösen Drucks jeweils mit vorgegebenen Grenzwerten verglichen werden. Bei den charakteristischen Werten, die mit den vorgegebenen Grenzwerten verglichen werden, handelt es sich also nicht um Werte, die aus dem arteriellen bzw. venösen Druck zu einem vorhergehenden Zeitpunkt und zu einem nachfolgendem Zeitpunkt berechnet worden sind. Allein die Summen- und Differenzbildung und der Vergleich mit vorgegebenen Grenzwerten reicht grundsätzlich aus, um zwischen einem möglichen fehlerhaften Zustand im extrakorporalen Kreislauf und einem Fehlalarm zu unterscheiden, der durch Patientenbewegungen sowie Viskositäts- und Blutdruckschwankungen des Patienten hervorgerufen werden kann.

Grundsätzlich wäre es auch möglich, das erfindungsgemäße Verfahren mit nur einem der beiden charakteristischen Werte, d.h. entweder der Summe oder der Differenz aus arteriellem oder venösem Druck, zu betreiben. Dabei könnten aber nicht alle Fehlalarme als solche erkannt werden, die Anzahl der Fehlalarme ließe sich aber um etwa die Hälfte reduzieren.

Bei der erfindungsgemäßen Vorrichtung wird von den arteriellen und venösen Drucksensoren Gebrauch gemacht, die in den bekannten Blutbehandlungsvorrichtungen ohnehin vorhanden sind. Somit beschränkt sich die maschinenseitige Änderung zur Implementierung des Schutzsystems lediglich auf eine Modifikation der automatisierten Auswertung vorhandener Sensordaten. Folglich kann das erfindungsgemäße Verfahren sehr einfach im Rahmen einer Softwareänderung implementiert werden.

Die vorgegebenen Grenzwerte bilden vorzugsweise ein Grenzwertfenster mit einem vorgegebenen oberen und unteren Grenzwert. Ein möglicher fehlerhafter Zustand wird dann erkannt, wenn die Alarm-Bedingung erfüllt ist, dass sowohl die Summe des arteriellen und venösen Drucks als auch die Differenz des arteriellen und venösen Drucks außerhalb des vorgegebenen Grenzwertfensters liegen. Wenn hingegen lediglich die Summe oder die Differenz des gemessenen arteriellen und venösen Drucks die Grenzen des Alarmfensters unter- bzw. überschreiten, wird angenommen, dass ein Fehlalarm vorliegt.

Für den Vergleich der Summe des arteriellen und venösen Drucks und der Differenz des arteriellen und venösen Drucks kann grundsätzlich das gleiche Grenzwertfenster herangezogen werden. Vorzugsweise werden aber unterschiedliche Grenzwertfenster angenommen, um die Alarmschwellen an die jeweiligen Therapieanforderungen individuell anpassen zu können. Die Alarmfenster mit einer Breite A bzw. einer Breite B können symmetrisch oder asymmetrisch um den aktuellen Summen- bzw. Differenzwert gesetzt werden.

Eine besonders bevorzugte Ausführungsform sieht eine weitere Überprüfung vor, um mit höherer Sicherheit einen fehlerhaften Zustand erkennen zu können. Auf einen wahrscheinlichen fehlerhaften Zustand wird dann geschlossen, wenn in n aufeinander folgenden Messungen jeweils die Alarm-Bedingung erfüllt ist, dass sowohl die Summe des arteriellen und venösen Drucks als auch die Differenz des arteriellen und venösen Drucks außerhalb der vorgegebenen Grenzwertfenster liegen.

Das erfindungsgemäße Verfahren und die erfindungsgemäße Vorrichtung können auch mit anderen Verfahren und Vorrichtungen zur Erkennung eines fehlerhaften Zustands bei einer extrakorporalen Blutbehandlung kombiniert werden. Damit kann die Sicherheit des Überwachungssystems noch weiter erhöht werden.

Für den Fall, dass ein fehlerhafter Zustand vorliegt, wird vorzugsweise ein akustischer und/oder optischer Alarm gegeben. Darüber hinaus kann der Blutfluss im extrakorporalen Blutkreislauf unterbrochen werden, um einen Blutverlust zu vermeiden. Eine Unterbrechung des Blutflusses ist bei den bekannten Vorrichtungen zur extrakorporalen Blutbehandlung dadurch möglich, dass die im extrakorporalen Kreislauf angeordnete Blutpumpe angehalten und/oder ein im extrakorporalen Kreislauf angeordnetes Sicherheitsventil, beispielsweise eine Schlauchklemme, geschlossen wird.

Eine Überwachung des extrakorporalen Blutkreislaufs kann nicht nur in Vorrichtungen zur Hämodialyse, Hämofiltration und Hämodiafiltration erfolgen, sondern auch in den bekannten Zellseparatoren, in denen das Blut eines Spenders in einem extrakorporalen Blutkreislauf einer Zentrifugation unterworfen und dabei in seine Bestandteile getrennt wird.

Im Folgenden wird das erfindungsgemäße Verfahren zur Überwachung eines extrakorporalen Blutkreislaufs sowie eine Vorrichtung zur extrakorporalen Blutbehandlung mit einer Einrichtung zur Überwachung des extrakorporalen Blutkreislaufs unter Bezugnahme auf die Zeichnungen anhand eines Ausführungsbeispiels näher erläutert.

Es zeigen:

Figur 1     der arterielle und venöse Druck als Funktion der Behandlungszeit während einer Bewegungsphase des Patienten,

Figur 2     der arterielle und venöse Druck sowie die Summe und Differenz des arteriellen und venösen Drucks als Funktion der Behandlungszeit während einer Bewegungsphase des Patienten,

Figur 3     eine Tabelle zur Veranschaulichung der Ursachen von Fehlalarmen

Figur 4     ein Ausführungsbeispiel einer Vorrichtung zur extrakorporalen Blutbehandlung mit einer Einrichtung zur Überwachung des extrakorporalen Blutkreislaufs in vereinfachter schematischer Darstellung und

Figur 5     einen Flussplan zur Beschreibung der Funktionsweise der Überwachungseinrichtung.

Der im arteriellen und venösen Zweig des extrakorporalen Blutkreislaufs gemessene arterielle Druck und venöse Druck setzen sich jeweils aus dem dynamischen Druck im extrakorporalen Kreislauf, der durch den Fluss der Blutpumpe erzeugt wird, und dem dynamischen Druck im Gefäßzugang des Patienten zusammen.

Der dynamische Druck im extrakorporalen Kreislauf ist eine Funktion des extrakorporalen Blutflusses sowie der Summe der Flusswiderstände im extrakorporalen Kreislauf. Da sich arterieller und venöser Flusswiderstand aufgrund der unterschiedlichen Geometrie der durchströmten Komponenten unterscheiden, ist die Summe des arteriellen und venösen Drucks, nachfolgend als $P_s$ bezeichnet, ebenfalls eine Funktion des Blutflusses. In der Regel wird bei den bekannten Blutreinigungsverfahren die Förderrate $Q_B$ der Blutpumpe auf einen festen Wert eingestellt. Somit ist die Summe der Flusswiderstände im extrakorporalen Kreislauf bei konstanter Viskosität des Blutes ebenfalls konstant.

Der dynamische Druck im Gefäßzugang des Patienten, im Folgenden Fisteldruck genannt, ist ebenfalls eine Funktion der Blutviskosität, des systemischen Blutdrucks sowie der systemischen vaskulären Flusswiderstände. Der Fisteldruck ist somit ein patientenspezifischer Parameter und hängt zusätzlich ab von der Art des Gefäßzugangs, der Viskosität des Blutes sowie dem Gefäßsystem, das den Gefäßzugang mit Blut versorgt. In Analogie zum dynamischen Druck im Extrakorporalsystem führt eine Änderung des Fisteldrucks beispielsweise durch Blutdruckschwankungen, Viskositätsschwankungen und Lageänderungen des Patienten zur Änderung sowohl des arteriellen als auch des venösen Druckwerts im extrakorporalen Kreislauf.

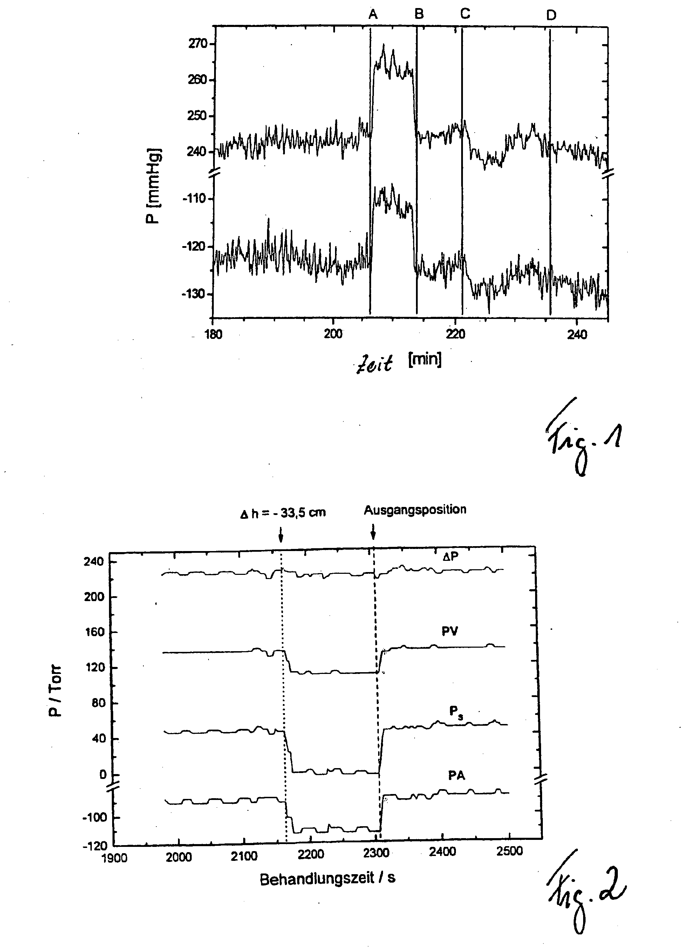

Figur 1 zeigt exemplarisch den Einfluss einer horizontalen Lageänderung des Patienten während einer Hämodialysebehandlung auf das Ausgangssignal der im arteriellen und venösen Zweig des extrakorporalen Kreislaufs vorgesehenen Drucksensoren. Im Zeitintervall von t=206-213 Minuten wurde das Patientenbett um ca. 20 cm nach oben gefahren. Deutlich ist eine symmetrische arterielle und venöse Druckerhöhung um ca. 15 mm Hg auf Grund der veränderten hydrostatischen Gleichgewichtsverhältnisse erkennbar. Bei falscher Wahl der Überwachungsfenster um den momentanen extrakorporalen Druck, würde diese für den Patienten völlig ungefährliche Lageänderung zu einem Fehlalarm führen, wenn die Überwachung des extrakorporalen Blutkreislaufs allein auf dem Vergleich des arteriellen und/oder venösen Drucks mit vorgegebenen Grenzwerten beruhen würde. Im Zeitintervall von 222-237 Minuten sind die Ausgangssignale der Drucksensoren zu erkennen, wenn der Patient den punktierten Fistelarm zum Essen verwendet. Auch hier ist eine symmetrische Druckänderung erkennbar. In beiden Fällen ist die Differenz des arteriellen und venösen Drucks, nachfolgend ΔP genannt, konstant, weshalb angenommen wird, dass es sich bei einem Alarm um einen Fehlalarm handelt.

Figur 2 zeigt das Verhalten des arteriellen und venösen Drucks bei einem konstanten effektiven Blutfluss $Q_B$, wenn sich die Lage des Patienten relativ zu den extrakorporalen Drucksensoren um - 33,5 cm ändert. Dies ist beispielsweise der Fall, wenn sich der Patient hinlegt oder seine Liege herunterfährt. Hierbei reduzieren sich die arteriellen und venösen Druckwerte um den Betrag der hydrostatischen Druckdifferenz, die ca. 0,75 Torr pro cm Höhendifferenz zwischen Drucksensor und Fistel beträgt. Die Zeitpunkte der Lageänderungen sind in Figur 2 mit gepunkteten Linien angegeben. Da sich bei einer Lageänderung des Patienten der arterielle und venöse Druck im extrakorporalen Kreislauf um den gleichen Wert ändern, bleibt die Differenz ΔP konstant. Hingegen sinkt die Summe $P_s$ des arteriellen und venösen Drucks um den doppelten Betrag der hydrostatischen Druckdifferenz. Dieses Verhalten zeigt sich analog bei einem Blutdruckabfall während der Behandlung. Auch hier sinken gleichfalls arterieller und venöser Druck im extrakorporalen Kreislauf um den gleichen Betrag. Somit ist deutlich erkennbar, dass bei getrennter Auswertung der extrakorporalen Drucksignale Fehlalarme während der Behandlung mit hoher Wahrscheinlichkeit auftreten, die mit dem erfindungsgemäßen Verfahren bzw. der erfindungsgemäßen Vorrichtung vermieden werden können.

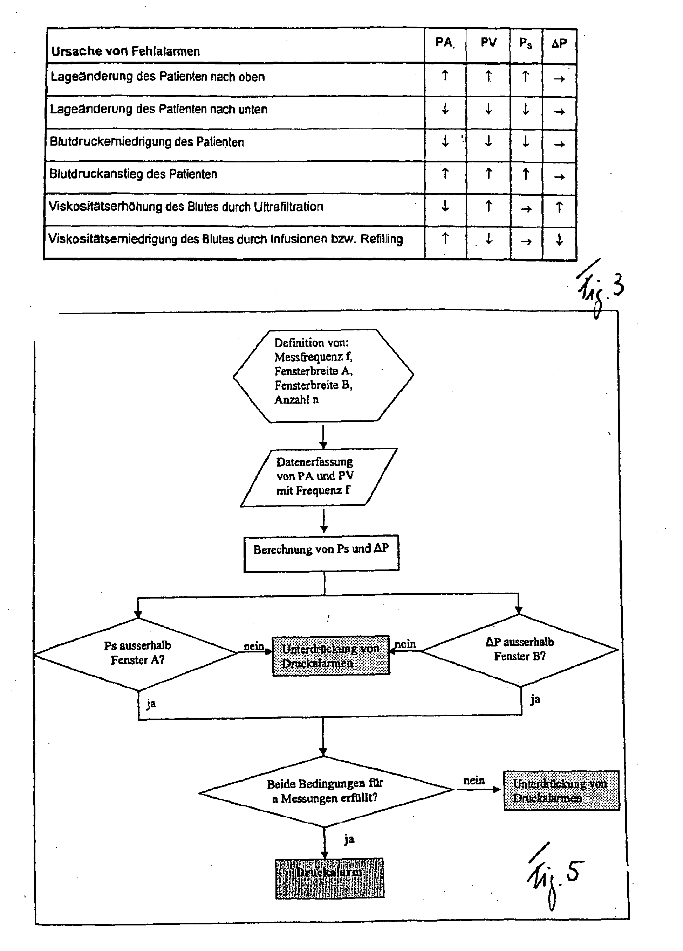

Die Tabelle in Figur 3 fasst die Ursachen der häufigsten Fehlalarme bei einer Lageänderung des Patienten, einer Blutdruckerniedrigung und einer Viskositätserhöhung qualitativ zusammen und zeigt den Trend der arteriellen und venösen Druckänderungen für die obigen Fälle.

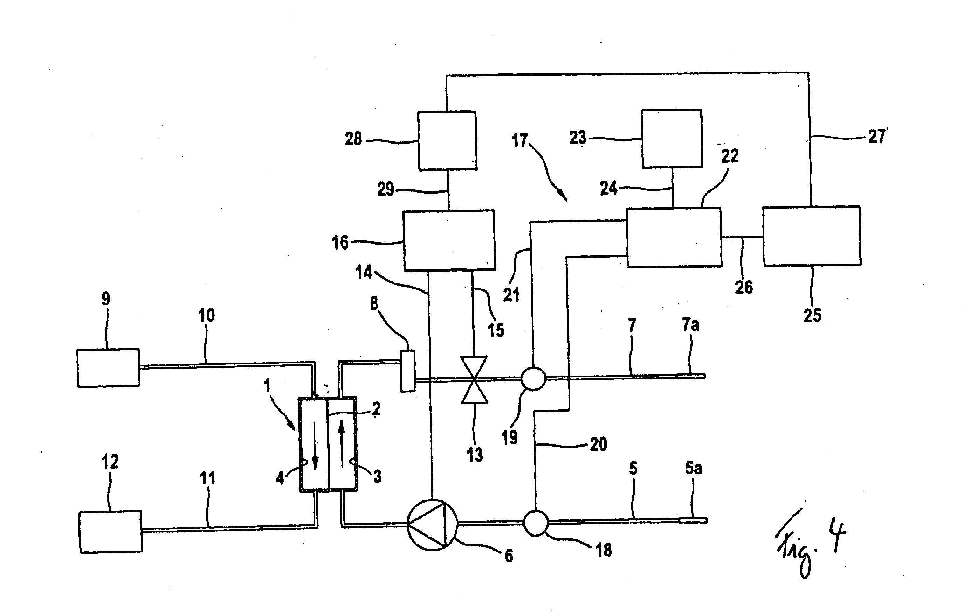

Figur 4 zeigt eine vereinfachte schematische Darstellung einer Dialysevorrichtung mit einer Einrichtung zur Überwachung des extrakorporalen Blutkreislaufs, die nachfolgend im Einzelnen beschrieben wird.

Die Dialysevorrichtung weist als Blutbehandlungseinrichtung einen Dialysator 1 auf, der durch eine semipermeable Membran 2 in eine Blutkammer 3 und eine Dialysierflüssigkeitskammer 4 unterteilt ist. An dem Einlass der Blutkammer ist eine arterielle Blutleitung 5 angeschlossen, in die eine peristaltische Blutpumpe 6 geschaltet ist. Stromab der Blutkammer 3 führt eine venöse Blutleitung 7 von dem Auslass der Blutkammer zu dem Patienten. In die venöse Blutleitung 7 ist eine Tropfkammer 8 geschaltet. An den Enden der arteriellen und venösen Blutleitung 5,7 sind Kanülen 5a, 7a angeschlossen, die jeweils in ein entsprechendes arterielles bzw. venöses Blutgefäß des Patienten gestochen werden. Die arterielle und venöse Blutleitung sind Bestandteile eines als Disposible ausgebildeten Schlauchleitungssystems, das den extrakorporalen Blutkreislauf bildet.

In einer Dialysierflüssigkeitsquelle 9 wird frische Dialysierflüssigkeit bereitgestellt. Von der Dialysierflüssigkeitsquelle 9 führt eine Dialysierflüssigkeitszuführleitung 10 zu dem Eingang der Dialysierflüssigkeitskammer 4 des Dialysators 1, während eine Dialysierflüssigkeitsabfuhrleitung 11 von dem Ausgang der Dialysierflüssigkeitskammer zu einem Abfluss 12 führt.

Die Dialysevorrichtung kann noch über weitere Komponenten, z.B. eine Bilanziereinrichtung und eine Ultrafiltrationseinrichtung etc., verfügen, die der besseren Übersichtlichkeit halber aber nicht dargestellt sind.

Zur Unterbrechung des Blutflusses ist an der venösen Blutleitung 7 stromab der Tropfkammer 8 eine Absperrklemme 13 vorgesehen, die elektromagnetisch betätigt wird. Die arterielle Blutpumpe 6 und die venöse Absperrklemme 13 werden über Steuerleitungen 14, 15 von einer Steuereinheit 16 angesteuert.

Die Einrichtung 17 zur Überwachung des Gefäßzuganges weist einen den Druck in der arteriellen Blutleitung 5 überwachenden arteriellen Drucksensor 18 und einen den Druck in der venösen Blutleitung 7 überwachenden venösen Drucksensor 19 auf. Die Messwerte der Drucksensoren 18, 19 werden über Datenleitungen 20, 21 an eine Recheneinheit 22 übertragen. In einer Speichereinheit 23, die über eine Datenleitung 24 mit der Recheneinheit 22 verbunden ist, werden die bei der Berechnung anfallenden Zwischenergebnisse gespeichert.

Zur Auswertung der für den fehlerhaften Zustand im extrakorporalen Blutkreislauf charakteristischen Werte weist die Überwachungseinrichtung 17 eine Auswerteinheit 25 auf, die über eine Datenleitung 26 mit der Recheneinheit 22 verbunden ist. Die Auswerteinheit 25 ist über eine Steuerleitung 27 an eine Alarmeinheit 28 angeschlossen, die wiederum über eine Steuerleitung 29 mit der Steuereinheit 16 verbunden ist.

Nachfolgend wird die Funktionsweise der Überwachungseinrichtung 17 unter Bezugnahme auf die Figuren 4 und 5 im Einzelnen beschrieben.

Zunächst werden die für die Auswertung erforderlichen Parameter festgelegt. Hierzu werden der obere und untere Grenzwert eines ersten Grenzwertfensters A und der obere und untere Grenzwert eines zweiten Grenzwertfensters B festgelegt. Die Grenzwerte zur Festlegung der Grenzwertfenster A, B können manuell eingegeben oder von der Überwachungseinrichtung als in der Speichereinheit 23 abgelegte Werte vorgegeben werden. Darüber hinaus werden die Anzahl n der Messungen und die Messfrequenz f vorgegeben.

Während der Dialysebehandlung werden mittels der Drucksensoren 18, 19 der arterielle und venöse Druck $P_{art}$, $P_{ven}$ mit der Messfrequenz f fortlaufend in aufeinander folgenden Zyklen von jeweils n aufeinander folgenden Messungen erfasst. Die Recheneinheit 22 berechnet in jedem Messzyklus nach jeder Messung 1, 2, 3... n die Summe $P_s$ des venösen und arteriellen Drucks und die Differenz $\Delta P$ zwischen dem venösen und arteriellen Druck, der mit den Drucksensoren gemessen wird. Zum Berechnen der Summe und der Differenz von arteriellem und venösen Druck verfügt die Recheneinheit über Mittel zum Bilden der Summe bzw. Differenz. $P_s$ und $\Delta P$ berechnen sich nach den folgenden Gleichungen:

$$P_s = P_{ven} + P_{art}$$

$P_{art}$: Messwert des arteriellen Drucks im extrakorporalen Kreislauf.

$P_{ven}$: Messwert des venösen Drucks im extrakorporalen Kreislauf.

$$\Delta P = P_{ven} - P_{art}$$

Nach jeder Messung 1, 2, 3...,n überprüft die Auswerteinheit 25, ob $P_s$ innerhalb oder außerhalb des Grenzwertfensters A liegt und $\Delta P$ innerhalb oder außerhalb des Grenzwertfensters B liegt. Für den Fall, dass $P_s$ oder $\Delta P$ innerhalb des Grenzwertfensters A bzw. B liegt, d.h. $P_s$ kleiner als der obere Grenzwert und größer als der untere Grenzwert des Grenzwertfensters A oder $\Delta P$ kleiner als der obere Grenzwert und größer als der untere Grenzwert des Grenzwertfensters B sind, schließt die Auswerteinheit 25 darauf, dass ein fehlerhafter Zustand im extrakorporalen Kreislauf nicht vorliegt. Demnach werden alle Druckalarme unterdrückt. Hierbei kann die Empfindlichkeit durch die Wahl der Breite der Alarmfenster A und B vorgegeben werden.

Für den Fall, dass sowohl $P_s$ außerhalb des Grenzwertfensters A als auch $\Delta P$ außerhalb des Grenzwertfensters B liegt, schließt die Auswerteinheit 25 darauf, dass ein möglicher fehlerhafter Zustand im extrakorporalen Kreislauf vorliegt.

Zur Reduzierung der Störanfälligkeit des Schutzsystems, beispielsweise bei kurzseitigen artifiziellen Druckschwankungen wird aber nicht nur eine Messung in einem Messzyklus, sondern n aufeinander folgenden Messungen für die Auswertung herangezogen.

Die Überwachungseinrichtung 17 führt während der Blutbehandlung in aufeinander folgenden Schritten jeweils n aufeinander folgende Messungen mit einer Frequenz f durch, um den arteriellen und venösen Druck zu messen. Für den Fall, dass für n Messungen sowohl $P_s$ als auch $\Delta P$ außerhalb des Grenzwertfensters A bzw. B liegen, schließt die Auswerteinheit 25 darauf, dass der mögliche fehlerhafte Zustand ein wahrscheinlicher fehlerhafter Zustand im extrakorporalen Kreislauf ist. Wenn beide Bedingungen für n aufeinander folgende Messungen erfüllt sind, gibt die Auswerteinheit 25 daher über die Steuerleitung 27 ein Alarmsignal an die Alarmeinheit 28, die einen akustischen und/oder optischen Alarm gibt.

Weiterhin gibt die Alarmeinheit 28 über die Steuerleitung 29 ein Alarmsignal an die Steuereinheit 16, die daraufhin die Blutpumpe 6 stoppt und die venöse Absperrklemme 13 schließt.

Wenn die beiden Bedingungen für n aufeinander folgende Messungen in einem Messzyklus jedoch nicht erfüllt sind, werden alle Druckalarme unterdrückt. Bei der Auswertung ist es möglich, einzelne Messungen zu unterdrücken, wenn die Messwerte als nicht repräsentative Ausreißer erscheinen.

**Patentansprüche**

1. Verfahren zur Überwachung eines extrakorporalen Blutkreislaufs während einer extrakorporalen Blutbehandlung, bei der Blut von einem arteriellen Gefäßzugang über einen arteriellen Zweig eines extrakorporalen Blutkreislaufs in eine Blutbehandlungseinrichtung strömt und aus der Blutbehandlungseinrichtung über einen venösen Zweig des extrakorporalen Kreislaufs zu einem venösen Gefäßzugang strömt, wobei der arterielle Druck im arteriellen Zweig und der venöse Druck im venösen Zweig des extrakorporalen Kreislaufs gemessen und aus der Summe und/oder der Differenz von Messwerten des arteriellen und venösen Drucks charakteristische Werte berechnet werden, die zur Erkennung von fehlerhaften Zuständen im extrakorporalen Kreislauf ausgewertet werden,
**dadurch gekennzeichnet,**
**dass** während der Blutbehandlung fortlaufend die Summe $P_s$ des zu dem selben Zeitpunkt gemessenen arteriellen und venösen Drucks $P_{art}$, $P_{ven}$ und/oder die Differenz $\Delta P$ des zu dem selben Zeitpunkt gemessenen arteriellen und venösen Drucks $P_{art}$, $P_{ven}$ jeweils mit vorgegebenen Grenzwerten verglichen werden.

2. Verfahren nach Anspruch 1, **dadurch gekennzeichnet, dass** geprüft wird, ob die Alarm-Bedingung erfüllt ist, dass die Summe $P_s$ des arteriellen und venösen Drucks und/oder die Differenz $\Delta P$ des arteriellen und venösen Drucks außerhalb vorgegebener Grenzen liegen, wobei auf einen möglichen fehlerhaften Zustand dann geschlossen wird, wenn die Alarm-Bedingung erfüllt ist.

3. Verfahren nach Anspruch 2, **dadurch gekennzeichnet, dass** die vorgegebenen Grenzwerte vorgegebene Grenzwertfenster A und/oder B mit einem oberen und unteren Grenzwert bilden, wobei geprüft wird, ob die Alarm-Bedingung erfüllt ist, dass die Summe $P_s$ des arteriellen und venösen Drucks und/oder die Differenz $\Delta P$ des arteriellen und venösen Drucks außerhalb der vorgegebenen Grenzwertfenster A bzw. B liegen.

4. Verfahren nach Anspruch 2 oder 3, **dadurch gekennzeichnet, dass** eine vorgegebene Anzahl n von aus zu aufeinander folgenden Zeitpunkten $t_1$, $t_2$, $t_3$, ..., $t_n$ gemessenen arteriellen und venösen Drükken, berechneten Summen $P_s$ und/oder Differenzen $\Delta P$ ausgewertet werden, wobei auf einen fehlerhaften Zustand geschlossen wird, wenn für die vorgegebene Anzahl n von Summen und/oder Differenzen, die Alarm-Bedingung erfüllt ist.

5. Verfahren nach einem der Ansprüche 1 bis 4, **dadurch gekennzeichnet, dass** bei Feststellung eines fehlerhaften Zustands im extrakorporalen Kreislauf ein Alarm gegeben wird.

6. Verfahren nach einem der Ansprüche 1 bis 5, **dadurch gekennzeichnet, dass** bei Feststellung eines fehlerhaften Zustands im extrakorporalen Kreislauf der Blutfluss im extrakorporalen Kreislauf unterbrochen wird.

7. Vorrichtung zur extrakorporalen Blutbehandlung mit einer arteriellen Blutleitung (5) eines extrakorporalen Blutkreislaufs, die an einem Ende mit einem Einlass einer Blutbehandlungseinrichtung (1) verbunden ist und an dem anderen Ende mit einem arteriellen Anschluss (5a) für einen Gefäßzugang versehen ist, einer venösen Blutleitung (7) des extrakorporalen Blutkreislaufs, die an einem Ende mit dem Auslass der Blutbehandlungseinrichtung (1) verbunden ist und an dem anderen Ende mit einem venösen Anschluss (7a) für den Gefäßzugang versehen ist, und
einer Einrichtung zur Überwachung des extrakorporalen Blutkreislaufs mit
einem den Druck in der arteriellen Blutleitung (5) überwachenden arteriellen Drucksensor (18) und einen den Druck in der venösen Blutleitung (7) überwachenden venösen Drucksensor (19),
einer Recheneinheit (22) zum Berechnen von charakteristischen Werten aus der Summe und/oder der Differenz von Messwerten des arteriellen und venösen Drucks, und einer
Auswerteinheit (25) zum Auswerten der charakteristischen Werte, um einen fehlerhaften Zustand im extrakorporalen Kreislauf zu erkennen,
**dadurch gekennzeichnet, dass** die Auswerteinheit (25) derart mit der Recheneinheit (22) zusammenwirkt und die Auswerteinheit (25) derart ausgebildet ist, dass während der Blutbehandlung fortlaufend die Summe $P_s$ des zu dem selben Zeitpunkt gemessenen arteriellen und venösen Drucks $P_{ven}$, $P_{art}$ und/oder die Differenz $\Delta P$ des zu dem selben Zeitpunkt gemessenen arteriellen und venösen Drucks $P_{ven}$, $P_{art}$ jeweils zur Erkennung von fehlerhaften Zuständen im extrakorporalen Kreislauf mit vorgegebenen Grenzwerten verglichen werden.

8. Vorrichtung nach Anspruch 7, **dadurch gekennzeichnet, dass** die Auswerteinheit (22) Mittel zum Prüfen der Alarm-Bedingung für einen möglichen fehlerhaften Zustand aufweist, dass die Summe $P_s$ des arteriellen und venösen Drucks $P_{art}$, $P_{ven}$ und/oder die Differenz $\Delta P$ des arteriellen und venösen Drucks $P_{art}$, $P_{ven}$ außerhalb vorgegebener Grenzen liegen, wobei auf einen möglichen fehlerhaften Zustand dann geschlossen wird, wenn die Alarm-Bedingung erfüllt ist.

9. Vorrichtung nach Anspruch 8, **dadurch gekennzeichnet, dass** die vorgegebenen Grenzwerte Grenzwertfenster A und/oder B mit einem vorgegebenen oberen und unteren Grenzwert bilden, und die Auswerteinheit (25) Mittel zum Vergleichen der Summe $P_s$ des arteriellen und venösen Drucks $P_{art}$, $P_{ven}$ und/oder der Differenz $\Delta P$ des arteriellen und venösen Drucks mit dem oberen und unteren Grenzwert der vorgegebenen Grenzwertfenster A bzw. B aufweist.

10. Vorrichtung nach Anspruch 9, **dadurch gekennzeichnet, dass** die Auswerteinheit (25) derart mit der Recheneinheit (22) zusammenwirkt, dass eine vorgegebene Anzahl n von aus zu aufeinander folgenden Zeitpunkten gemessenen arteriellen und venösen Drücken berechnete Summen $P_s$ und/oder Differenzen $\Delta P$ ausgewertet werden und auf einen fehlerhaften Zustand geschlossen wird, wenn die vorgegebene Anzahl n von Summen und/oder Differenzen, die Alarm-Bedingung erfüllt.

11. Vorrichtung nach einem der Ansprüche 7 bis 10, **dadurch gekennzeichnet, dass** eine Alarmeinheit (28) vorgesehen ist, die bei Feststellung eines fehlerhaften Zustandes im extrakorporalen Kreislauf einen Alarm gibt.

12. Vorrichtung nach einem der Ansprüche 7 bis 11, **dadurch gekennzeichnet, dass** Mittel (6, 13) zum Unterbrechen des Blutflusses im extrakorporalen Kreislauf bei Feststellung eines fehlerhaften Zustandes im extrakorporalen Kreislauf vorgesehen sind.

**Claims**

1. A method for monitoring an extracorporeal blood circuit during extracorporeal blood treatment, in which the blood from an arterial vascular access flows via an arterial branch of an extracorporeal blood circuit into a blood treatment device and flows from the blood treatment device via a venous branch of the extracorporeal blood circuit to a venous vascular access, wherein the arterial pressure in the arterial branch and the venous pressure in the venous branch of the extracorporeal blood circuit are measured, and characteristic values are calculated from the sum and/or difference of measured values of the arterial and venous pressure, and are evaluated to detect faulty states in the extracorporeal circuit, **characterized in that** the sum $P_s$ of the simultaneously measured arterial and venous pressure $P_{art}$, $P_{ven}$ and/or the difference $\Delta P$ of the simultaneously measured arterial and venous pressure $P_{art}$, $P_{ven}$ are in each case continuously compared with specified limit values during the blood treatment.

2. The method as claimed in claim 1, **characterized in that** a check is carried out as to whether the alarm condition is fulfilled, that the sum $P_s$ of the arterial and venous pressure and/or the difference $\Delta P$ of the arterial and venous pressure lie outside specified limits, wherein it is concluded that a possible faulty state exists when the alarm condition is fulfilled.

3. The method as claimed in claim 2, **characterized in that** the specified limit values form specified limit value windows A and/or B with an upper and lower limit value, wherein a check is carried out as to whether the alarm condition is fulfilled, that the sum $P_s$ of the arterial and venous pressure and/or the difference $\Delta P$ of the arterial and venous pressure lie outside the specified limit value windows A and B respectively.

4. The method as claimed in claim 2 or 3, **characterized in that** a specified number n of sums $P_s$ and/or differences $\Delta P$, which are calculated from arterial and venous pressures measured at successive points in time $t_1$, $t_2$, $t_3$, ..., $t_n$, are evaluated, wherein it is concluded that a faulty state exists when the alarm condition is fulfilled for the specified number n of sums and/or differences.

5. The method as claimed in one of claims 1 to 4, **characterized in that** an alarm is given when a faulty state is determined in the extracorporeal circuit.

6. The method as claimed in one of claims 1 to 5, **characterized in that** the blood flow in the extracorporeal circuit is interrupted when a faulty state is determined in the extracorporeal circuit.

7. A device for extracorporeal blood treatment having an arterial blood line (5) of an extracorporeal blood circuit, which, at one end, is connected to an inlet of a blood treatment device (1) and, at the other end, is provided with an arterial connection (5a) for a vascular access,
a venous blood line (7) of the extracorporeal blood circuit, which at one end is connected to the outlet of the blood treatment device (1) and, at the other end, is provided with a venous connection (7a) for the vascular access, and
a device for monitoring the extracorporeal blood circuit having
an arterial pressure sensor (18), which monitors the pressure in the arterial blood line (5), and a venous pressure sensor (19), which monitors the pressure in the venous blood line (7),
an arithmetic unit (22) for calculating characteristic values from the sum and/or the difference of measured values of the arterial and venous pressure, and an
evaluation unit (25) for evaluating the characteristic values in order to detect a faulty state in the extracorporeal circuit,
**characterized in that** the evaluation unit (25) works together with the arithmetic unit (22) and the evaluation unit (25) is designed in such a way that the sum $P_s$ of the simultaneously measured arterial and venous pressure $P_{ven}$, $P_{art}$ and/or the difference $\Delta P$ of the simultaneously measured arterial and venous pressure $P_{ven}$, $P_{art}$ are in each case continuously compared with specified limit values during the blood treatment in order to detect faulty states in the extracorporeal circuit.

8. The device as claimed in claim 7, **characterized in**

**that** the evaluation unit (22) has means for checking the alarm condition for a possible faulty state, that the sum $P_s$ of the arterial and venous pressure $P_{art}$, $P_{ven}$ and/or the difference $\Delta P$ of the arterial and venous pressure $P_{art}$, $P_{ven}$ lie outside specified limits, wherein it is concluded that a possible faulty state exists when the alarm condition is fulfilled.

9. The device as claimed in claim 8, **characterized in that** the specified limit values form limit value windows A and/or B with a specified upper and lower limit value, and the evaluation unit (25) has means for comparing the sum $P_s$ of the arterial and venous pressure $P_{art}$, $P_{ven}$ and/or the difference $\Delta P$ of the arterial and venous pressure with the upper and lower limit value of the specified limit value windows A and B respectively.

10. The device as claimed in claim 9, **characterized in that** the evaluation unit (25) works together with the arithmetic unit (22) in such a way that a specified number n of sums $P_s$ and/or differences $\Delta P$, which are calculated from arterial and venous pressures measured at successive points in time, are evaluated, and it is concluded that a faulty state exists when the specified number n of sums and/or differences fulfils the alarm condition.

11. The device as claimed in one of claims 7 to 10, **characterized in that** an alarm unit (28), which gives an alarm when a faulty state is established in the extracorporeal circuit, is provided.

12. The device as claimed in one of claims 7 to 11, **characterized in that** means (6, 13) are provided for interrupting the blood flow in the extracorporeal circuit when a faulty state is determined in the extracorporeal circuit.

**Revendications**

1. Procédé de surveillance d'un circuit de circulation sanguine extracorporel pendant un traitement extracorporel du sang, lors duquel du sang circule d'un accès vasculaire artériel à un dispositif de traitement du sang via une branche artérielle d'un circuit de circulation sanguine extracorporel et circule du dispositif de traitement du sang à un accès vasculaire veineux via une branche veineuse du circuit de circulation extracorporel, dans lequel la pression artérielle dans la branche artérielle et la pression veineuse dans la branche veineuse du circuit de circulation extracorporel sont mesurées et à partir de la somme et/ou de la différence de valeurs de mesure des pressions artérielle et veineuse, des valeurs caractéristiques sont calculées et sont analysées pour reconnaître des états défectueux dans le circuit de circulation extracorporel, **caractérisé en ce que** pendant le traitement du sang, la somme $P_s$ des pressions artérielle et veineuse $P_{art}$, $P_{ven}$ mesurées au même instant et/ou la différence $\Delta P$ des pressions artérielle et veineuse $P_{art}$, $P_{ven}$ mesurées au même instant, sont comparées continûment avec des valeurs limites prédéterminées.

2. Procédé selon la revendication 1, **caractérisé en ce qu'**il est vérifié si la condition d'alarme est remplie, selon laquelle la somme $P_s$ des pressions artérielle et veineuse et/ou la différence $\Delta P$ des pressions artérielle et veineuse se situent en dehors de limites prédéterminées, dans lequel il est conclu à un possible état défectueux lorsque la condition d'alarme est remplie.

3. Procédé selon la revendication 2, **caractérisé en ce que** les valeurs limites prédéterminées forment des fenêtres de valeurs limites prédéterminées A et/ou B avec une valeur limite supérieure et une valeur limite inférieure, dans lequel il est vérifié si la condition d'alarme est remplie, selon laquelle la somme $P_s$ des pressions artérielle et veineuse et/ou la différence $\Delta P$ des pressions artérielle et veineuse se situent en dehors des fenêtres de valeurs limites prédéterminées A ou B.

4. Procédé selon la revendication 2 ou 3, **caractérisé en ce qu'**un nombre prédéterminé n de sommes $P_s$ et/ou de différences $\Delta P$ calculées à partir de pressions artérielle et veineuse mesurées à des instants successifs $t_1$, $t_2$, $t_3$, ...., $t_n$, sont analysées dans lequel il est conclu à un état défectueux, lorsque la condition d'alarme est remplie pour le nombre prédéterminé n de sommes et/ou de différences.

5. Procédé selon l'une quelconque des revendications 1 à 4, **caractérisé en ce qu'**une alarme est émise lors de la constatation d'un état défectueux dans le circuit de circulation extracorporel.

6. Procédé selon l'une quelconque des revendications 1 à 5, **caractérisé en ce que** le flux sanguin dans le circuit de circulation extracorporel est interrompu lors de la constatation d'un état défectueux dans le circuit de circulation extracorporel.

7. Dispositif de traitement extracorporel du sang avec une canalisation sanguine artérielle (5) d'un circuit de circulation sanguine extracorporel, qui est raccordée par une extrémité à une entrée d'un dispositif de traitement du sang (1) et qui est munie à l'autre extrémité d'un raccord artériel (5a) pour un accès vasculaire,
une canalisation sanguine veineuse (7) du circuit de circulation sanguine extracorporel, qui est raccordée par une extrémité à la sortie du dispositif de traitement du sang (1) et qui est munie à l'autre extrémité d'un raccord veineux (7a) pour l'accès vasculaire, et un dispositif de surveillance du circuit de circulation sanguine extracorporel, avec

un capteur de pression artériel (18) surveillant la pression dans la canalisation sanguine artérielle (5) et un capteur de pression veineux (19) surveillant la pression dans la canalisation sanguine veineuse (7), une unité de calcul (22) pour calculer des valeurs caractéristiques à partir de la somme et/ou de la différence de valeurs de mesure des pressions artérielle et veineuse, et une

unité d'analyse (25) pour analyser les valeurs caractéristiques, afin de reconnaître un état défectueux dans le circuit de circulation extracorporel, **caractérisé en ce que** l'unité d'analyse (25) coopère avec l'unité de calcul (22) et l'unité d'analyse (25) est configurée de telle manière que la somme $P_s$ des pressions artérielle et veineuse $P_{art}$, $P_{ven}$ mesurées au même instant et/ou la différence $\Delta P$ des pressions artérielle et veineuse $P_{art}$, $P_{ven}$ mesurées au même instant soient comparées en continu, pendant le traitement du sang, avec des valeurs limites prédéterminées afin de reconnaître des états défectueux dans le circuit de circulation extracorporel.

8. Dispositif selon la revendication 7, **caractérisé en ce que** l'unité d'analyse (25) comporte des moyens pour vérifier la condition d'alarme pour un possible état défectueux, selon laquelle la somme $P_s$ des pressions artérielle et veineuse $P_{art}$, $P_{ven}$ et/ou la différence $\Delta P$ des pressions artérielle et veineuse $P_{art}$, $P_{ven}$ se situent en dehors de limites prédéterminées, dans lequel il est conclu à un état défectueux lorsque la condition d'alarme est remplie.

9. Dispositif selon la revendication 8, **caractérisé en ce que** les valeurs limites prédéterminées forment des fenêtres de valeurs limites A et/ou B avec une valeur limite supérieure et une valeur limite inférieure, et l'unité d'analyse (25) comporte des moyens pour comparer la somme $P_s$ des pressions artérielle et veineuse $P_{art}$, $P_{ven}$ et/ou la différence $\Delta P$ des pressions artérielle et veineuse avec la valeur limite supérieure et inférieure des fenêtres de valeurs limites prédéterminées A ou B.

10. Dispositif selon la revendication 9, **caractérisé en ce que** l'unité d'analyse (25) coopère avec l'unité de calcul (22) de telle manière qu'un nombre prédéterminé n de sommes $P_s$ et/ou de différences $\Delta P$ calculées à partir de pressions artérielles et veineuses mesurées à des instants successifs soient analysées et qu'il est conclu à un état défectueux lorsque le nombre prédéterminé n de sommes et/ou de différences remplissent la condition d'alarme.

11. Dispositif selon l'une quelconque des revendications 7 à 10, **caractérisé en ce qu'**il est prévu une unité d'alarme (28), qui émet une alarme lors de la constatation d'un état défectueux dans le circuit de circulation extracorporel.

12. Dispositif selon l'une quelconque des revendications 7 à 11, **caractérisé en ce qu'**il est prévu des moyens (6, 13) pour interrompre le flux sanguin dans le circuit de circulation extracorporel lors de la constatation d'un état défectueux dans le circuit de circulation extracorporel.

*Fig. 1*

*Fig. 2*

| Ursache von Fehlalarmen | PA | PV | $P_s$ | $\Delta P$ |
|---|---|---|---|---|
| Lageänderung des Patienten nach oben | ↑ | ↑ | ↑ | → |
| Lageänderung des Patienten nach unten | ↓ | ↓ | ↓ | → |
| Blutdruckerniedrigung des Patienten | ↓ | ↓ | ↓ | → |
| Blutdruckanstieg des Patienten | ↑ | ↑ | ↑ | → |
| Viskositätserhöhung des Blutes durch Ultrafiltration | ↓ | ↑ | → | ↑ |
| Viskositätserniedrigung des Blutes durch Infusionen bzw. Refilling | ↑ | ↓ | → | ↓ |

Fig. 3

Definition von:
Messfrequenz f,
Fensterbreite A,
Fensterbreite B,
Anzahl n

Datenerfassung
von PA und PV
mit Frequenz f

Berechnung von Ps und $\Delta P$

Ps ausserhalb Fenster A?   nein   Unterdrückung von Druckalarmen   nein   $\Delta P$ ausserhalb Fenster B?

ja                                                                                        ja

Beide Bedingungen für n Messungen erfüllt?   nein   Unterdrückung von Druckalarmen

ja

Druckalarm

Fig. 5

Fig. 4

EP 2 040 776 B1

13

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 1584339 A2
- WO 03002174 A1
- DE 10159620 C1
- US 20020190863 A1

**In der Beschreibung aufgeführte Nicht-Patentliteratur**

- **WIKLUND L ; HÖK B ; STÄHL K ; JORDE-BY-JÖNSSON A.** Postanaesthesia monitoring revisited: Frequency of true and false alarms from different monitoring devices. *J. Clin Anesth,* 1994, vol. 6, 182-188